# EUROPEAN PATENT APPLICATION

(11) **EP 3 922 167 A1**
(43) Date of publication of application: **15.12.2021**
(21) Application number: 21179029.0
(22) Date of filing: 11.06.2021
(51) Int. Cl.: A61B 3/14, G02B 3/14, G03B 15/03

(54) **CAMERA AND METHOD FOR OPERATING A CAMERA**

(30) Priority: 12.06.2020 DE 102020115648; 07.12.2020 DE 102020132517
(71) Applicant: Optotune AG, 8953 Dietikon (CH)
(72) Inventor: NIEDERER, David Andreas, 5024 Küttigen (CH); ASCHWANDEN, Manuel, 6319 Allenwinden (CH)
(74) Representative: Schulz Junghans Patentanwälte PartGmbB

(57) **Abstract**

The present invention relates to a camera comprising a sensor element, and an aperture, wherein
- the aperture comprises an opaque element having a plurality of through holes, a plurality of tunable lenses and an illumination device,
- wherein tunable lenses are assigned to the through holes respectively, and
- the illumination device is arranged on a side of the aperture, facing away from the sensor element.

## Description

The present invention relates to a camera and a method for operating a camera.

International patent application WO 2013/007612 A1 describes a light field camera for fundus imaging comprising a lens array. The focal length of the lenses of the lens array is constant.

The camera described herein is arranged to capture images or videos. In particular the camera is a funds camera. For example, the camera is a non-mydriatic fundus camera, which enables retinal photography without pharmacologic dilation of the pupil. Non-mydriatic imaging is effective through pupils with a diameter of at least 2.0 mm. The fundus camera makes use of the retina's reflective properties to show details and store images that are superior to slit lamps and other commonly-used tools. Advantageously, the fundus camera does not require pupil dilation in the majority of cases and is painless for patients.

In the following embodiments and further aspects of the present invention are described.

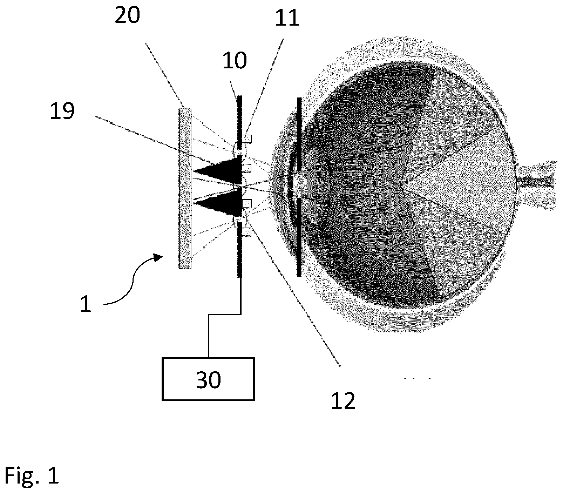

As shown in figure 1, the camera 1 comprises an image sensor 20. The image sensor may have a resolution of 1080 mega pixel having a size of 1 inch times 1.33 inch. In particular, the image sensor has at least 15 million pixels, preferably at least 30 million pixels.

The camera 1 comprises an aperture 10 having an opaque element 13 with multiple through holes 14 and a plurality of tunable lenses 12. Each tunable lens is arranged to one of the through holes. In particular a tunable lens arranged in each through hole.

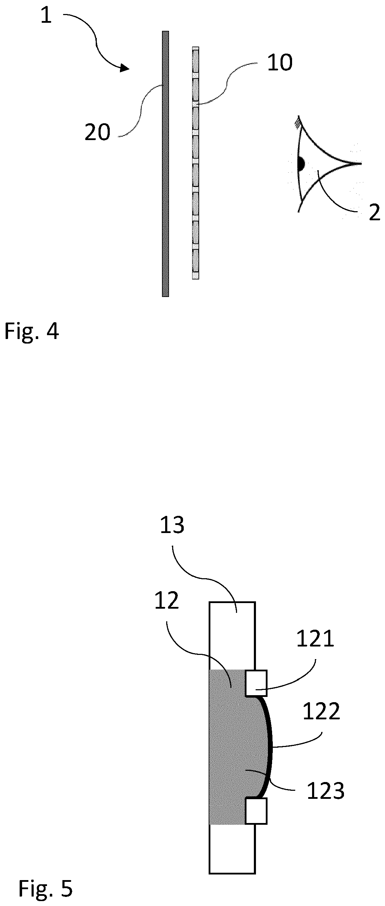

As show in figure 5, the tunable lenses may comprise a liquid volume 123 and a membrane 122, wherein the optical power of the tunable lenses is altered by changing the curvature of the membrane. The curvature of the membrane is altered by moving a liquid in the liquid volume. In particular, the tuning state of the tunable lenses is altered independently. For example, each tunable lens comprises a lens shaper 121, which extends circumferentially around the membrane 122. The tunable lens is tuned by changing the position of the shaper along the optical axis of the respective lens and/or by increasing the pressure of the liquid the liquid volume.

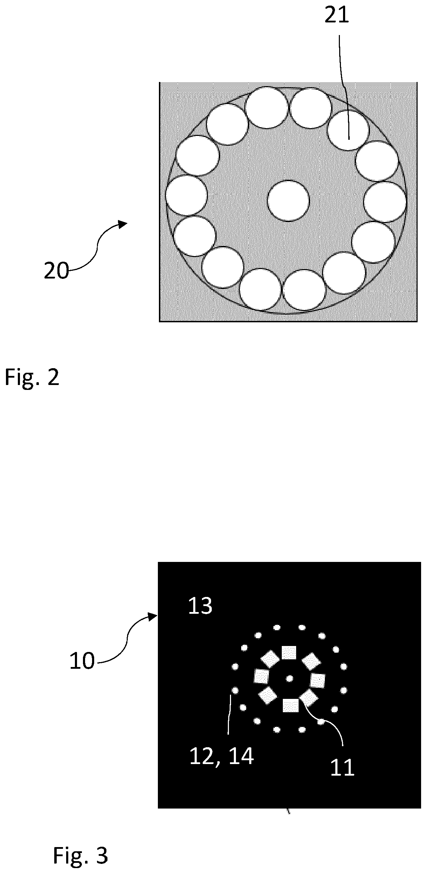

A shown in figure 3, the aperture comprises an illumination device 11, which is arranged to illuminate a region of interest The region of interest is arranged on a side of the aperture, facing away from the sensor element. The camera is arranged to capture light which is emitted by the illumination device and which is reflected in the region of interest. The camera is arranged to capture an image of a human eye 2 arranged within the region of interest.

Figure 4 shows a camera 1 in a schematic sectional view. The camera is a fundus camera and comprises a sensor element 20, and an aperture 10, wherein the aperture comprises an opaque element having a plurality of through holes, a plurality of tunable lenses and an illumination device. The illumination device is not depicted in figure 4. Tunable lenses are assigned to the through holes respectively, and the tunable lenses are arranged in the through holes of the aperture respectively. The tunable lenses are arranged at the corner points of an imaginary repetitive hexagonal line pattern, at the corner points of an imaginary repetitive rectangular line pattern or along the lines of an imaginary concentric circular pattern.

The illumination device is arranged on a side of the aperture, facing away from the sensor element. The tunable lenses are arranged in a common plane. The illumination device is arranged to illuminate a region of interest on a side of the aperture facing away from the sensor element. In particular, the camera is arranged to capture images of an eye which is arranged in the region of interest. The sensor is arranged to capture a portion of the light reflected in the region of interest, wherein the portion of the reflected light passes through the tunable lenses and the through holes.

For example, each tunable lens comprises a tunable surface or at least two tunable surfaces, wherein the refractive power is tunable by changing the curvature of the tunable surface or the tunable surfaces. The tunable surface may be formed by a membrane which is arranged adjacent to a volume filled with a liquid, wherein the curvature of the tunable surface is altered by moving the liquid. The volume of the tunable lenses is arranged in the through holes of the aperture and the aperture delimits the volume at least partially. In particular, the plurality of tunable lenses comprises a common membrane which extends continuously over the plurality of through holes. The refractive power of the tunable lenses is tunable in a range -40 diopters to +30 diopters.

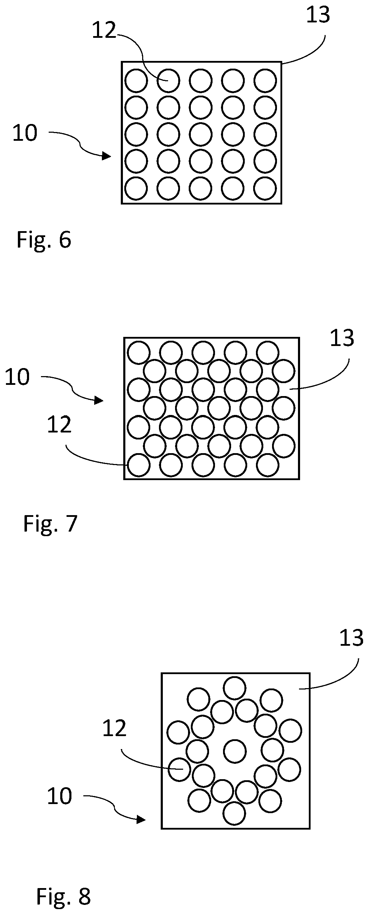

As shown in figure 8, the through holes 14 and the tunable lenses 12 may be arranged in a concentric pattern. Alternatively, the through holes 14 and the tunable lenses 12 may be arranged at the corner points of an imaginary repetitive hexagonal line pattern (see figure 7) or at the corner points of an imaginary repetitive rectangular line pattern (see figure 6).

In particular, the aperture comprises blocking elements 19. The blocking elements are arranged between the opaque element and the image sensor. The blocking elements are made from an opaque material. As shown in figure 2, the image sensor comprises multiple subsections 21, wherein each subsection is assigned to one of the through holes. Said subsections of the sensor may overlap with each other. In particular, the blocking elements are arranged to prevent crosstalk between adjacent subsections of the image sensor.

For example, the camera has a single field of view range of at least 200°. In particular, eye position guidance allows to enlarge the field of view. An image captured by eye position guidance has a field of view of at least 240°.

According to one embodiment, the camera comprises a focus unit 30. The focus unit is arranged to tune the tunable lenses to a dedicated tuning state or to define a time span in which an image is captured by means of the sensor element. In particular the focus unit is arranged to identify patterns. Thereby the focus unit may identify the retina and the choroid of a human eye. In particular the focus unit is arranged to control the camera, so that images of the choroid and the retina are captured automatically, in particular essentially simultaneously.

According to one embodiment the camera is arranged for external eye and anterior segment imaging.

According to one embodiment, the camera has an automatic mode, wherein the focus unit automatically controls the focus plane in which an image is captured. Furthermore, the camera may have a manual mode, wherein the user defines in which focus plane an image is captured.

According to one embodiment, the optical power of the tunable lenses is tunable in a range from -13 diopters to +12 diopters.

According to one embodiment, the camera is arranged to capture an image in less than 1 second. In particular said image may consist of multiple sub-images, wherein the sub-images are taken at different tuning states of the tunable lenses. The image may be taken without considering light in the wavelength range of red light. In particular, the illumination unit does not emit light in a wavelength rang of red light or the aperture or the image senor comprise a sensor, blocking light in the red wavelength range from being captured. In particular the image captured may be a monochromatic image. In particular, the camera is arranged to detect autofluorescence. Autofluorescence is the natural emission of light by biological structures such as mitochondria and lysosomes when they have absorbed light, and is used to distinguish the light originating from artificially added fluorescent markers (fluorophores). The most commonly observed autofluorescencing molecules are NADPH and flavins; the extracellular matrix can also contribute to autofluorescence because of the intrinsic properties of collagen and elastin. Generally, proteins containing an increased amount of the amino acids tryptophan, tyrosine and phenylalanine show some degree of autofluorescence.

The tunable lenses of the camera work similar to the refractive optical element described in connection with the display unit disclosed in the German patent application 10 2020 115 648.0 which content is hereby incorporated by reference.

According to a first aspect, the camera comprises a sensor element, and an aperture, wherein
- the aperture comprises an opaque element having a plurality of through holes, a plurality of tunable lenses and an illumination device,
- wherein tunable lenses are assigned to the through holes respectively, and
- the illumination device is arranged on a side of the aperture, facing away from the sensor element.

According to a second aspect the camera is a fundus camera.

According to a third aspect, the the illumination device is arranged to illuminate a region of interest on a side of the aperture facing away from the sensor element, wherein the sensor is arranged to capture a portion of the light reflected in the region of interest, wherein the portion of the reflected light passes through the tunable lenses and the through holes.

According to a fourth aspect the illumination device comprises an LED which is arranged between two of the through holes and in particular which is fixedly attached to the aperture.

According to a fifth aspect, the tunable lenses are arranged in a common plane.

According to a sixth aspect, the tunable lenses are arranged at the corner points of an imaginary repetitive hexagonal line pattern, at the corner points of an imaginary repetitive rectangular line pattern or along the lines of an imaginary concentric circular pattern.

According to a seventh aspect the tunable lenses are arranged in the through holes of the aperture respectively.

According to an eighth aspect, the tunable lens comprises a tunable surface or at least two tunable surfaces, wherein the refractive power is tunable by changing the curvature of the tunable surface or the tunable surfaces.

According to a ninth aspect, the tunable surface is formed by a membrane which is arranged adjacent to a volume filled with a liquid, wherein the curvature of the tunable surface is altered by moving the liquid.

According to a tenth aspect, the volume of the tunable lenses is arranged in the through holes of the aperture and the aperture delimits the volume at least partially.

According to an eleventh aspect, the plurality of tunable lenses comprises a common membrane which extends continuously over the plurality of through holes.

According to a twelfth aspect refractive power of the tunable lenses is tunable in a range -40 diopters to +30 diopters.

According to a thirteenth aspect, the a method for operating a camera comprising a sensor element and an aperture having a plurality of through holes and a plurality of tunable lenses is provided, wherein the refractive optical element (12) has a refractive power, which is tunable, comprising the steps of,
a1) tuning the tunable lenses synchronously in an oscillating manner;
b1) capturing an image by means of the sensor element in a definable time span, when the refractive power of the tunable lenses is within a dedicated range, or
a2) tuning the tunable lenses to a dedicated refractive power, wherein the refractive power of at least two of the tunable lenses differs;
b) capturing an image by means of the sensor element

According to a fourteenth aspect the sensor element comprises multiple subregions, wherein the aperture images different sections of the region of interest in the subregions, and the method comprises the method steps a1) and b1), wherein the time span in which each subregion captures the image is selected independently.

According to a fifteenth aspect the camera is a fundus camera which is arranged to capture an image of a mamal's eye, wherein the method comprises the method steps a1) and b1) and at least one subregion captures an image of the retina and at least one subregion captures an image of the choroid during one period of the oscillation of the oscillation of the tunable lenses, or the method comprises the method steps a2) and b2) and at least one subregion captures an image of the retina and at least one subregion captures an image of the choroid simultaneously.

According to a sixteenth aspect in method step a1) the refractive power is tuned with a frequency of at least 45Hz, in particular at least 60Hz, and an amplitude of at least 5 diopters in particular at least 10 diopters.

According to a seventeenth aspect the refractive power is set to an offset value, and the refractive power oscillates around said offset value in method step a1).

According to an eighteenth aspect, the offset value is between -40 and +30 diopters and the offset value depends on the ametropia of the person using the camera.

According to a nineteenth aspect, the camera comprises a focus unit, which is arranged to automatically adjust the tuning state of the tunable lenses and/or which is arranged to automatically select a time span.

According to a twentieth aspect the camera is a fundus camera, the focus unit is arranged to identify the retina and the choroid, the focus unit is arranged to adjust the tuning state of the tunable lenses and/or the time span to capture an image so that at least one of the subregions captures an image of the retina and at least one of the subregions captures an image of the choroid.

Finally, in the following, aspects of the present invention and embodiments of these aspects are stated as items. These items may also be formulated as claims.

Item 1: Camera comprising a sensor element, and an aperture, wherein
- the aperture comprises an opaque element having a plurality of through holes, a plurality of tunable lenses and an illumination device,
- wherein tunable lenses are assigned to the through holes respectively, and
- the illumination device is arranged on a side of the aperture, facing away from the sensor element.

Item 2: Camera according to the preceding item, wherein the camera is a fundus camera.

Item 3: Camera according to the preceding item, wherein
- the illumination device is arranged to illuminate a region of interest on a side of the aperture facing away from the sensor element, wherein
- the sensor is arranged to capture a portion of the light reflected in the region of interest, wherein the portion of the reflected light passes through the tunable lenses and the through holes.

Item 4: Camera according to one of the preceding items, wherein the illumination device comprises an LED which is arranged between two of the through holes and in particular which is fixedly attached to the aperture.

Item 5: Camera according to one of the preceding items, wherein the tunable lenses are arranged in a common plane.

Item 6: Camera according to the preceding item, wherein the tunable lenses are arranged at the corner points of an imaginary repetitive hexagonal line pattern, at the corner points of an imaginary repetitive rectangular line pattern or along the lines of an imaginary concentric circular pattern.

Item 7: Camera according to one of the preceding items, wherein the tunable lenses are arranged in the through holes of the aperture respectively.

Item 8: Camera according to one of the preceding items, wherein each tunable lens comprises a tunable surface or at least two tunable surfaces, wherein
the refractive power is tunable by changing the curvature of the tunable surface or the tunable surfaces.

Item 9: Camera according to the preceding item, wherein the tunable surface is formed by a membrane which is arranged adjacent to a volume filled with a liquid, wherein the curvature of the tunable surface is altered by moving the liquid.

Item 10: Camera according to the preceding item, wherein the volume of the tunable lenses is arranged in the through holes of the aperture and the aperture delimits the volume at least partially.

Item 11: Camera according to the preceding items 9 or 10, wherein the plurality of tunable lenses comprises a common membrane which extends continuously over the plurality of through holes.

Item 12: Camera according to one of the preceding items, wherein the refractive power of the tunable lenses is tunable in a range -40 diopters to +30 diopters.

Item 13: Method for operating a camera comprising a sensor element and an aperture having a plurality of through holes and a plurality of tunable lenses, wherein the refractive optical element has a refractive power, which is tunable, comprising the steps of,
a1) tuning the tunable lenses synchronously in an oscillating manner;
b1) capturing an image by means of the sensor element in a definable time span, when the refractive power of the tunable lenses is within a dedicated range, or
a2) tuning the tunable lenses to a dedicated refractive power, wherein the refractive power of at least two of the tunable lenses differs;
b) capturing an image by means of the sensor element.

Item 14: Method according to the preceding item, wherein the sensor element comprises multiple subregions, wherein the aperture images different sections of the region of interest in the subregions, and
the method comprises the method steps a1) and b1), wherein the time span in which each subregion captures the image is selected independently.

Item 15: Method according to the preceding item, wherein the camera is a fundus camera which is arranged to capture an image of a mamal's eye, wherein
- the method comprises the method steps a1) and b1) and at least one subregion captures an image of the retina and at least one subregion captures an image of the choroid during one period of the oscillation of the oscillation of the tunable lenses, or
- the method comprises the method steps a2) and b2) and at least one subregion captures an image of the retina and at least one subregion captures an image of the choroid simultaneously.

Item 16: Method according to the preceding item, wherein in method step a1) the refractive power is tuned with a frequency of at least 45Hz, in particular at least 60Hz, and an amplitude of at least 5 diopters in particular at least 10 diopters.

Item 17: Method according to one of the preceding items, wherein the refractive power set to an offset value, and the refractive power oscillates around said offset value in method step a1).

Item 18: Method according to the preceding item, wherein the offset value is between -40 and +30 diopters and the offset value depends on the ametropia of the person using the camera.

Item 19: Method according to one of the preceding items, wherein the camera comprises a focus unit, which is arranged to automatically adjust the tuning state of the tunable lenses and/or which is arranged to automatically select a time span.

Item 20: Method according to the preceding item, wherein
- the camera is a fundus camera,
- the focus unit is arranged to identify the retina and the choroid,
- the focus unit is arranged to adjust the tuning state of the tunable lenses and/or the time span to capture an image so that at least one of the subregions captures an image of the retina and at least one of the subregions captures an image of the choroid.

### List of reference signs

- 1: Camera
- 2: Human eye
- 10: Aperture
- 11: Illumination unit
- 12: Tunable lens
- 13: Opaque element
- 14: Through hole
- 122: membrane
- 121: Lens shaper
- 123: Liquid volume
- 19: Blocking element
- 20: Image sensor
- 21: subsection
- 30: Focus unit

## Claims

1. Camera comprising a sensor element, and an aperture, wherein
- the aperture comprises an opaque element having a plurality of through holes, a plurality of tunable lenses and an illumination device,
- wherein tunable lenses are assigned to the through holes respectively, and
- the illumination device is arranged on a side of the aperture, facing away from the sensor element.

2. Camera according to the preceding claim, wherein the camera is a fundus camera.

3. Camera according to the preceding claim, wherein
- the illumination device is arranged to illuminate a region of interest on a side of the aperture facing away from the sensor element, wherein
- the sensor is arranged to capture a portion of the light reflected in the region of interest, wherein the portion of the reflected light passes through the tunable lenses and the through holes.

4. Camera according to one of the preceding claims, wherein the tunable lenses are arranged in a common plane.

5. Camera according to the preceding claim, wherein
the tunable lenses are arranged at the corner points of an imaginary repetitive hexagonal line pattern, at the corner points of an imaginary repetitive rectangular line pattern or along the lines of an imaginary concentric circular pattern.

6. Camera according to one of the preceding claims, wherein the tunable lenses are arranged in the through holes of the aperture respectively.

7. Camera according to one of the preceding claims, wherein each tunable lens comprises a tunable surface or at least two tunable surfaces, wherein
the refractive power is tunable by changing the curvature of the tunable surface or the tunable surfaces.

8. Camera according to one of the preceding claims, wherein the refractive power of the tunable lenses is tunable in a range -40 diopters to +30 diopters.

9. Method for operating a camera comprising a sensor element and an aperture having a plurality of through holes and a plurality of tunable lenses,
wherein the refractive optical element has a refractive power, which is tunable, comprising the steps of,
a1) tuning the tunable lenses synchronously in an oscillating manner;
b1) capturing an image by means of the sensor element in a definable time span, when the refractive power of the tunable lenses is within a dedicated range, or
a2) tuning the tunable lenses to a dedicated refractive power, wherein the refractive power of at least two of the tunable lenses differs;
b) capturing an image by means of the sensor element

10. Method according to the preceding claim, wherein the sensor element comprises multiple subregions, wherein the aperture images different sections of the region of interest in the subregions, and
the method comprises the method steps a1) and b1), wherein the time span in which each subregion captures the image is selected independently.

11. Method according to the preceding claim, wherein the camera is a fundus camera which is arranged to capture an image of a mamal's eye, wherein
- the method comprises the method steps a1) and b1) and at least one subregion captures an image of the retina and at least one subregion captures an image of the choroid during one period of the oscillation of the oscillation of the tunable lenses, or
- the method comprises the method steps a2) and b2) and at least one subregion captures an image of the retina and at least one subregion captures an image of the choroid simultaneously.

12. Method according to the preceding claim, wherein in method step a1) the refractive power is tuned with a frequency of at least 45Hz, in particular at least 60Hz, and an amplitude of at least 5 diopters in particular at least 10 diopters.

13. Method according to one of the preceding claims, wherein the refractive power set to an offset value, and the refractive power oscillates around said offset value in method step a1).

14. Method according to one of the preceding claims, wherein the camera comprises a focus unit, which is arranged to automatically adjust the tuning state of the tunable lenses and/or which is arranged to automatically select a time span.

15. Method according to the preceding claim, wherein
- the camera is a fundus camera,
- the focus unit is arranged to identify the retina and the choroid,
- the focus unit is arranged to adjust the tuning state of the tunable lenses and/or the time span to capture an image so that at least one of the subregions captures an image of the retina and at least one of the subregions captures an image of the choroid.
